Europäisches Patentamt

(19) European Patent Office

Office européen des brevets

(11) Publication number: **0 195 304**
A2

(12) # EUROPEAN PATENT APPLICATION

(21) Application number: 86102808.2

(22) Date of filing: 04.03.86

(51) Int. Cl.⁴: **C 12 N 9/96**
C 12 N 11/02, C 12 N 1/04
//C12P19/24

(30) Priority: 04.03.85 US 707773

(43) Date of publication of application:
24.09.86 Bulletin 86/39

(84) Designated Contracting States:
BE DE FR GB IT NL

(71) Applicant: THE DOW CHEMICAL COMPANY
2030 Dow Center Abbott Road P.O. Box 1967
Midland, MI 48640(US)

(72) Inventor: Cheng, Roberta C.
3873 Old Pine Trail
Midland Michigan 48640(US)

(72) Inventor: McCoy, Karen M.
2308 Carol Court
Midland Michigan 48640(US)

(72) Inventor: Houtchens, Robert A.
2008 Larel Lane
Midland Michigan 48640(US)

(72) Inventor: Moll, Norman G.
2563 Daniels Road
Sanford Michigan 48657(US)

(74) Representative: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel Möhlstrasse 22
D-8000 München 80(DE)

(54) Stabilization of intracellular enzymes.

(57) The subject invention concerns a process for stabilizing
intact or ruptured microbial cells having active enzymes
associated therewith. Specifically exemplified is a process
for stabilizing glucose isomerase producing cells of a
microorganism belonging to the genus *Ampullariella*. In the
invention process the whole or ruptured microbial cells are
contacted with a partially modified cationic polyelectrolyte,
e.g., a partially modified polyamine, to stabilize and floccu-
late the cells. It is critical that the polyelectrolyte be partially
modified; a fully modified or non-modified polyelectrolyte
will not give the surprising beneficial results of the invention
process. The flocculated cells are further stabilized by
encapsulation with a partially modified cationic polyelectro-
lyte. The encapsulation can be done prior to or after the
flocculated cells are cross-linked. The net effect is manifested
by a dramatic increase in the half-life of the enzyme, thus
leading to a more effective use of the immobilized enzyme.

# STABILIZATION OF INTRACELLULAR ENZYMES

Microbe-catalyzed processes are particularly useful in the production of a variety of chemicals known as fine or specialty chemicals.

Perhaps the most important commercial use of microbe-catalyzed processes is in the food industry. Exemplary of such processes is the production of high fructose corn syrup (HFCS) catalyzed by immobilized glucose isomerase. This process converts glucose to an approximately equimolar mixture of fructose and glucose; this mixture is referred to as HFCS.

Examples of other chemicals prepared by microbe-catalyzed processe are the L-amino acids, which are useful as food additives, in animal feed, and in medicinals. Though chemical synthesis may be simpler than fermentations at times to prepare amino acids, the chemical process almost always yields a racemic mixture of amino acids. This racemic mixture then has to be resolved to give the biologically-active L-amino acid. On the other hand, a microbe-catalyzed process will yield the L-amino acid predominantly.

32,805-F

Immobilization of the enzyme which catalyzes the many and varied microbe-catalyzed processes generally gives better yields of desired product and preserves enzyme integrity. Basically, immobilization is the conversion of enzymes from a water-soluble, mobile state to a water-insoluble, immobile state. The immobilization of the enzyme can be done while the enzyme is still in the living microbe (intracellular), or when the enzyme is in the cell-free state. The immobilization techniques will vary in accord with these two enzyme states. Thus, it should be appreciated that immobilization conditions effective for intracellular enzymes will not necessarily be appropriate for extracellular enzymes, and vice versa.

In accordance with this invention microbial cells having active enzymes associated therewith are stabilized by a process which comprises contacting said microbial cells (intact or ruptured) with a partially--modified cationic polyelectrolyte whereby the cells are flocculates and, if desired encapsulated. It is critical that the polyelectrolyte, e.g., a polyamine, be only partially modified in order to realize the surprising benefits of the process. For example, the half-life time of glucose isomerase-producing microbial cells, disclosed hereinafter, which were contacted with a partially modified polyamine, was estimated to be about 1484 hours. The control, wherein similar microbial cells were contacted with unmodified polyamine, gave a half-life of about 669 hours. Fully carboxymethylated polyethyleneimine (PEI) has also been tested. The preparation with fully carboxymethylated PEI had a half-life of about 310 hours. Partial

0195304

modification of the polyelectrolyte can be by carboxy-alkylation, or by phosphonoalkylation, wherein the alkyl is $-(CH_2)_n-$ wherein n=1-3 (preferably n=1), or $-(CHR)-$ $-(CH_2)_n-$ wherein R=methyl, ethyl, propyl, or isopropyl, and n=1 or 2.

Further stabilization of the immobilized enzyme is obtained by encapsulating the immobilized cells with protective layers of polymer(s). In general, encapsulation has been found to give a two-fold increase in enyzme half-life time. This added stabilization of enzyme is applicable for processes carried out in a pH range of 6 to 8.5, advantageously at pH 6 to 7.5. The advantages of operating the isomerization process at lower pH, which are well known to HFCS (High Fructose Corn Syrup) producers, are: (1) it minimizes pH adjustment before and after the isomerization, (2) it reduces by-products formation and thus reduces purification costs, and (3) purer HFCS can be obtained.

As used herein flocculation refers to a process which involves the use of polyelectrolytes to promote the aggregation of small particles in an aqueous medium. Flocculants have been used successfully to facilitate the isolation of microbial cells from fermentation broths. In the immobilization of cellular materials, which are usually present in a fermentation broth, flocculation is included in the process for the following reasons: 1) to simplify the isolation and dehydration of the cellular materials, and 2) additives may be added and coflocculated with the cellular materials to impart desirable properties to the immobilized enzyme preparations. (P.L. Busch

32,805-F

and W. Stumn in Environmental Science and Technology, 2, 49-53 (January 1968); and L. L. Gasner and D. I. C. Wang in Biotechnology and Bioengineering 12 873-887 (1970)).

To surround a particle with a membraneous envelope is encapsulation. In the field of enzymology, microencapsulation of enzyme or enzyme producing organisms has been used as meas of immobilization and enzyme stabilization. Typically, the enzyme is encapsulated in semipermeable membranes. The semipermeable membrane serves as a barrier to prevent the enzyme from leaking out to the substrate solution, and to keep the impurities from getting close to and accelerating the enzyme inactivation. (T. M. S. Chang, Science, 146, 524 (1964)). According to the present invention, the immobilized enzyme (IME) is encapsulated by dipping (or coating) the IME particles in a solution of e.g., a partially carboxymethylated PEI to form a thin membraneous envelope which is insolubilized upon cross-linking.

Cross-linking agents that may be employed in the practice of this invention include any difunctional and/or multi-functional reagent that is reactive towards amino groups, such as, aldehydes, for example, glutaraldehyde, glyoxal, dialdehyde starch and polyglutaraldehyde; isocyantes, for example, toluene--2,4-diisocyanate, hexamethylene-diisocyanate and diphenyl-methane-diisocyante; thioisocyanates, for example, hexamethylene-diisothiocyante; anhydrides, for example, polymethacrylic anhydride and poly-(ethylene-maleic anhydride); water soluble carbodiimides, for example, 1-cyclohexyl-3-(2-morpholinoethyl) carbodiimide and metho-o-toluene sulfonate-N,N'-dicyclohexylcarbodiimide; chloro-triazines, for example, cyanuric chloride; diazo compounds, for example, bis--diazobenzidine-3,3' -disulfonate and tetraazotized -O-dianisidine difluorobenzenes, for example, 1,3-di-fluoro-4,6-dinitrobenzene; epoxy compounds, for example, epichlorohydrin; phosgene derivatives, for example, ethyl chloroformate; and halogenoalkyl derivatives, for example, bromoacetylbromide.

This invention concerns a process to stabilize and flocculate, and, if desired, encapsulate, intact or ruptured microbial cells having active enzymes associated therewith.  The process is particularly useful wherein the microbial cells are in an immobilized system.  Though immobilization itself is viewed as a stabilization procedure for the intracellular enzyme, the subject process increases the stability factor, as measured by the half-life of the particular enzyme, and also, advantageously, flocculates the intact or ruptured microbial cells.  The net result is an improved process for using the enzyme in a microbial-catalyzed process.

The process is specifically exemplified herein by being applied to a process for converting glucose to fructose with immobilized glucose isomerase. Similar techniques can be used with microbial cells producing other enzymes.  Necessary modifications to accomodate the use of such other microbial cells are well within the skill of those in the microbiological enzyme art.

Glucose isomerase, can be produced by a large number of microbes as disclosed, e.g., in U.S. Patent

32,805-F

4,308,349, Col. 1, lines 26-32. The _Ampullariella_ species of this patent have been found to be especially good producers of glucose isomerase. Other glucose isomerase-producing microbes known to the art and available to the public also can be used for making the glucose isomerase used in the process of this invention.

The intact or ruptured microbial cell having an active enzyme associated therewith, is contacted with a partially-modified cationic polyelectrolyte. Advantageously, the modification can be done by carboxyalkylating the polyelectrolyte by processes well known in the art. See, e.g., U.S. Patent 3,424,790, which discloses a process for preparing carboxymethylated polyethyleneimine. The polyelectrolyte also can be partially modified by phosphonoalkylation. Phosphonoalkylation is a well-known art process. See, e.g., Westerback et al. J. Am. Chem. Soc. $\underline{87}$, 2567 (1965); Moedritzer et al. J. Org. Chem. $\underline{31}$, 1603 (1966). The alkyl in the above is $-(CH_2)_n-$ wherein n=1-3 (preferably n=1), or $-(CHR)-(CH_2)_n-$ wherein R=methyl, ethyl, propyl, or isopropyl, and n=1 or 2.

The amount of modification of the polyelectrolyte, e.g., carboxymethylation of the polyethyleneimine, can be varied from 0.1 to 1.0 equivalents of polyelectrolyte, preferably 0.25 to 0.5. This is accomplished by reacting a limited amount of chloroacetic acid (from 0.1 to 1.0 equivalents of the polyelectrolyte as measured by the total nitrogen content). Thus the fractional number preceding CM-PEI found in this application represents the stochiometric ratio of chloroacetic acid to the total nitrogen in PEI used in the synthesis.

32,805-F

Polyelectrolytes within the scope of the subject invention, which can be partially modified as disclosed above, are classified as cationic polyelectrolytes, for example, polyamines (primary, secondary, tertiary and quaternary amines); poly-aminoacids, for example, polylysine; cationic polyacrylamides, for example, polydimethylaminopropyl-methacrylamide; cationic poly(vinyl chloride), for example, poly(vinyl chloride) aminated with triethylene tetraamine; cationic copolymers, for example, styrene dimethylaminopropylmethacrylamide (50:50) copolymer; and cationic flocculants, for example, Purifloc C-31 (Trademark of The Dow Chemical Company.)

When used in an immobilized cell system, the partially-modified cationic polyelectrolyte can be incorporated into the immobilized system. Thus it is physically separated from the feed and product. This feature, advantageously, eliminates the need for removal of the partially-modified cationic polyelectrolyte from the final product. Also, since the partially--modified cationic polyelectrolyte is "built into" the immobilized system, no pretreatment of the feed is requried. This, then, minimizes operational cost in using the immobilized system.

Another advantageous aspect of the invention process can be shown when applied to immobilized glucose isomerase systems. The process has a wider operable pH range (pH 6 to 8.5) than either of the well-known commercial products as shown in Table I.

The partially-modified cationic polyelectrolyte (CM-PEI) stabilized immobilized enzyme system also is less sensitive to impurities present in the feed.

Encapsulation of the immobilized cells greatly enhances the stability of the enzyme. The encapsulation can be of the flocculated cells (particles) or the flocculated-cross-linked cells. Polymers which can be used to encapsulate the cells are the same as described herein for flocculating the microbial cells.

The polyelectrolyte used for encapsulation can be more highly modified than that used for flocculating the microbial cells. The modification can be from 0.2 to 1.0 equivalents of polyelectrolyte, preferably 0.4 to 0.75.

The immobilized enzyme catalyst is prepared in suitably sized particles, for example, cyclindrical particles, with a diameter of 500 to 840 microns, ranging in length from 0.1 to 0.2 inches ($^{\circ}$.254 to $^{\circ}$.508 cm), and being brown to dark brown in color. The particles have sufficient structural stability to allow continuous operation in column reactors for 2000 hours or longer.

A brief summary of the advantageous aspects of the invention process, as exemplified by partially carboxymethylated PEI is: (1) flocculates the microbes and enzyme very well; (2) contains groups, e.g., ($HN-CH_2-COOH$) groups, effective in scavenging impurities and/or metal ions present in the glucose feed stream; and (3) contains primary amine functionalities to allow covalent cross-linking of the flocculant with the cellular materials.

Following are examples that illustrate the process and products of the invention. All percentages are by weight and all solvent mixture proportions are by volume unless otherwise noted.

## Example 1 - Flocculation

A suspensin of whole cells of Ampullariella 3876, ATCC 31351, (500 ml; containing 3.31 percent of solid) was adjusted to pH 8.0 with 5 N potassium hydroxide. To this was added a partially carboxymethylated polyethyleneimine ([CM-PEI]; with CM to PEI ratio of 0.25, at pH 7.0 containing 0.019 equivalent of nitrogen/100 ml) with vigorous stirring for one minute, followed by gentle stirring for 5 more minutes. The flocculated cells were collected in an IEC chemical centrifuge basket (I.E. #1303, 5 inch dia. x 2½ inch depth 12.7 x 6.35 cm) (International Equipment Company, Needham Ht., Massachusetts) at 5200 rpm, and washed with 1500 ml of Milli-Q water (Trademark of Millipore Corp. Milford, Massachusetts, for highly purified water). The cell paste was then heat-treated at 70°C for 1 hour. The heart-treated cells (containing approximately 30 to 40 percent solid) were extruded through a 0.03 inch (0.762 mm) (I.D.) exit tubing on a French press (American Instrument Co., Silver Spring, Maryland) at 4000 psi (27,5788N/mm$^2$) and into 800 ml of acetone. After being dehydrated in the acetone bath for 1 hour, the extrudates were collected and further dried in a vacuum oven for 2-3 hours to remove the residual acetone, and then were pelletized in a Waring blender (3 x 5 second pulses), and sieved. The particles of 500-840 microns were used for cross-linking and testing.

## Cross-linking

Cell particles were weighed (7 grams) into 42 ml of glutaraldehyde solution (containing 4.2 ml of 25 percent glutaraldehyde, 16.8 ml of water and 21 ml of 0.2 M potassium phosphate) at pH 8.0. This was incubated at 25°C and 200 revolutions per minute (rpm) in a shaker bath for 1 hour. The resulting cross-linked particles were collected on a sintered glass (coarse) funnel and washed 3 times with Milli-Q water (3 x 200 ml) and dried in a vacuum oven at room temperature for 24 hours. The particles were again sieved and the 500-840 micron fraction was used for column studies. Glucose isomerase activity of the immobilized enzyme (IME), determined by batch assay in a substrate containing 5 percent glucose, 50 mM of $Mg^{++}$ and 1 mM of $Co^{++}$ in 0.25 M maleate buffer at pH 6.5 and 70°C, was 63± 3 GIU/g (1 GIU is defined as the glucose isomerase (GI) activity which catalyzes the isomerization of 1 micromole of glucose to fructose per minute under standard assay conditions).

Stability of half-life time (t 1/2) of the immobilized enzyme was determined in continuous up-flow column reactors (9x150 mm) at 60°C, with a flow rate of 30 ml/hour, and residence time of approximately 20 minutes. The substrate used for the study contained 50 percent (W/V) glucose, 3 mM $Mg^{++}$, similar to that used by high fructose corn syrup producers. Sodium azide (0.02 percent) was added as an antimicrobial. The half-life time of the 0.25 CM-PEI flocculated and cross--linked cells of Ampullariella 3876 at pH 8.2 was estimated to be 1484 hours, compared to t 1/2 of 669 hours for the control, which was prepared similarly, using PEI-600 (Cordova Chemical Company, Muskegon, Michigan), a polyethyleneimine product, as the flocculant. (See Table II)

Example 2

Whole cells of Ampullariella 3876 were flocculated with a CM-PEI (CM to PEI ratio of 0.5) and cross-linked with 15 percent glutaraldehyde as described above. The IME thus prepared had a t 1/2 of 958 hours.

The enzyme catalyst prepared in the above examples is dark brown in color, and cylindrical in shape. The particle size is bout 500 to 840 microns in diameter and >0.1 inch (0.254 cm) in length. It possesses sufficient physical stability to allow continuous operation for over 2000 hours in column reactors.

Example 3

Upon flocculating the whole cells of Ampullariella 3876 with a fully carboxymethylated PEI, and cross-linking with glutaraldehyde according to the procedure disclosed in Examples 1 and 2, there was obtained an immobilized glucose isomerase having inferior stability compared to the partially CM-PEI flocculated and cross-linked Ampullariella 3876 described in Examples 1 and 2. The t 1/2 was estimated to be 310 hours at pH 8.2.

Example 4

Whole cells of Ampullariella 3876 were flocculated with 6 percent flocculants (based on the total dry weight of the cellular material) listed

32,805-F                                    -11-

in Table III and cross-linked with 15 percent glutaraldehyde in the same manner as described in Example 1. Half-life time (summarized in Table III) of the immobilized cells of Ampullariella 3876 thus obtained were determined at pH 6.6 and 60°C in similar fashion to Example 1.

Example 5 - Encapsulation Prior to Cross-linking

Particles prepared as described in Example 1 in the flocculation section were soaked in a solution of partially modified CM-PEI (ratio of CM to N being 0.4), equivalent to 6 percent PEI per gram of cellular material. The bulk of the water was removed by evaporation and lyophilization overnight, and the encapsulated and flocculated cell particles were cross-linked with glutaraldehyde as follows:

Cross-linking -- Five grams of the encapsulated particles were weighed into 15 ml of a cross-linking solution containing 0.75 grams of glutaraldehyde in 0.1 M of potassium phosphate buffer at pH 8.0. This was incubated at 25°C and 200 rpm on a shaker for 1 hour. Upon completion, it was washed with Milli-Q water (3 x 100 ml) and dried thoroughly in a vacuum oven. The particles were again sieved and the fraction of 500-840 microns was used for stability study in the column reactor.

Stability study -- Four grams of the immobilized glucose isomerase (IMGI) preparation was immersed in the glucose substrate (50 percent cerelose dextrose [W/V] containing 3 mM $MgSO_4$, and 0.02 percent sodium azide at pH 6.6). After 2 hours, it was packed into a Teflon column (0.5 x 12 in (1.27 x 30.48 cm)),

32,805-F                                    -12-

and the half-life time (t 1/2) of the IMGI was deter-minded in a continuous upflow process with the 50 percent glucose solution, assuming pseudo-first order kinetics for the enzyme inactivation. Results are shown in Table IV.

Example 6 - Encapsulation after Cross-linking

A sample (4.0 grams) of 0.25 CM-PEI flocculated and glutaraldehyde cross-linked Ampullariella, prepared as described in Example 1, was encapsulated with a partially carboxymethylated PEI (CM: N=0.4; equivalent to 0.24 g of PEI), as described in Example 5. The encapsulated IMGI was cross-linked with 5 percent glutaraldehyde in 0.1 M potassium phosphate buffer at pH 8.0 for 30 min. The stabilities of the treated as well as the untreated IMGI were tested in the usual manner. The t 1/2 of the treated was 1018 hours compared to 350 for the untreated in the 50 percent glucose substrate at pH 6.6 and 60°C.

The effect of partially carboxymethylated PEI encapsulation on the stability of immobilized cells of Ampullariella 3876 is shown in Table IV.

Example 7 - Encapsulation of Commercially Available Enzyme

A sample (3 grams) of Novo Sweetzyme Q (Trademark of Novo Industries A/S, Bagsvaerd, Denmark, for glucose isomerase preparation) was immersed in 12 ml of a partially carboxymethylated PEI (CM: N=0.4) solution containing 0.18 grams of PEI at pH 7. The excess liquid was evaporated under nitrogen, and the bulk of the water was removed by lyophilization overnight. The Sweetzyme Q particles encapsulated with CM-PEI were then cross-linked with glutaraldehyde according to the procedure described in Example 1, except that only 5 percent glutaraldehyde was used, and the reaction time was shortened to 30 minutes. Stability of the CM-PEI encapsulated Sweetzyme Q was determined in continuous upflow column reactors (9 x 150 mm) at 60°C, with a flow rate of 30 ml/hour, and residence time of approximately 20 minutes. The substrate used for the study contains 50 percent (W/V) glucose, 3 mM MG$^{++}$, similar to that used by high fructose corn syrup producers. Sodium azide (0.02 percent) is added as an antimicrobial. The t 1/2 in the 50 percent glucose substrate at pH 6.6 and 60°C was 291 hours, compared with 70-115 hours for the untreated Sweetzyme Q.

Example 8 - Encapsulation of Another Commercially Available Enzyme

A sample (2 grams) of Taka-Sweet (Trademark of Miles Laboratories, Inc., Elkhart, Indiana, for glucose isomerase preparation) was treated with a partially carboxymetylated PEI solution (CM: N=0.4), equivalent to 0.12 grams of PEI, according to the previous example. The t 1/2 determined in a column reactor was 725 hours compared to 333-406 for the untreated Taka-Sweet in 50 percent glucose substrate at pH 6.6 and 60°C.

Example 9

D-glucose is converted to D-fructose in an aqueous medium by contacting the D-glucose with glucose isomerase, prepared as described in the above examples.

Operable temperature and pH ranges generally vary
from 45°C to 85°C and from pH 6 to 8.5, respectively.
The produced D-fructose is recovered by procedures
well known in the art.

TABLE I


Half-Life Time of Immobilized Glucose
Isomerase at Various pH's and 60°C


| IME | 6.6 | t 1/2 at pH 7.4 (hrs) | 8.2 |
|---|---|---|---|
| 0.625 CM-PEI Encapsulated and 0.25 CM-PEI Flocculated | | | |
| Amp* | 1042 | --- | 1540 |
| 0.25 CM-PEI Flocculated Amp* | 352 | 1248 | 1484 |
| Novo Sweetzyme Q** | 147 | 452 | 1490 |
| G. B. Maxazyme*** | 130 | 325 | 1143 |

Substrate was 50 percent glucose (W/V) containing 3 mM $Mg^{++}$ and 0.02 percent sodium azide.

*Ampullariella 3876

**Trademark of Novo Industries A/S Bagsvaerd, Denmark

***Trademark of Gist-Brocades NV, Holland

TABLE II

Stability of Partially Carboxymethylated Poly-
ethyleneimine Flocculated and Glutaraldehyde
Cross-linked Cells of <u>Ampullariella</u> at pH 8.2
and 60°C

| Ration of CM to Nitrogen | First t 1/2, Hrs |
|---|---|
| 0 | 669 |
| 0.25 | 1484 |
| 0.5 | 1181 |
| 1.0 | 983 |
| 2.0 | 310 |

TABLE III

Stability Comparison of Immobilized <u>Ampullariella</u> 3876 with Various Flocculants at pH 6.6 and 60°C

| Flocculants | First t 1/2, Hrs |
|---|---|
| PEI | 156 |
| 0.25 CM-PEI | 352 |
| 0.4 CM-PEI | 357 |
| 0.5 GM-PEI | 683 |
| Purifloc C-31 | 221 |
| 0.25 CM-Purifloc C-31 | 329 |
| 0.15 CM-Purifloc C-31 | 298 |

## TABLE IV

Effect of Partially Carboxymethylated PEI Encapsulation
on the Stability (Measured at t 1/2) of Immobilized
Cells of <u>Ampullariella</u> 3876
at pH 6.6 and 60°C

| Flocculant | Encapsulation | Half-Life, Hrs |
|---|---|---|
| 6% 0.25 CM-PEI | None | 352 |
| 6% 0.25 CM-PEI | 6% 0.625 CM-PEI | 914 |
| 6% 0.2 CM-PEI | 6% 0.4 CM-PEI | 855 |
| 6% 0.2 CM-PEI | 6% 0.625 CM-PEI | 849 |
| 6% 0.2 CM-PEI | 6% 0.5 CM-PEI | 679 |
| 6% 0.25 CM-PEI | 9% 0.625 CM-PEI | 928 |
| 6% 0.25 CM-PEI | 18% 0.625 CM-PEI | 1042 |
| 6% Purifloc C-31* | None | 221 |
| 6% Purifloc C-31 | 6% 0.4 CM-PEI | 667 |
| 6% Purifloc C-31 | 12% 0.4 CM-PEI | 1087 |
| 6% Purifloc C-31 | 18% 0.4 CM-PEI | 1047 |
| 6% 0.25 CM Purifloc C-31 | None | 329 |
| 6% 0.25 CM Purifloc C-31 | 6% 0.625 CM-PEI | 1131 |
| 6% 0.15 CM Purifloc C-31 | None | 298 |
| 6% 0.15 Purifloc C-31 | 6% 0.625 CM-PEI | 819 |

*Trademark of The Dow Chemical Company

Note:  All the immobilized cells were cross-linked
with glutaraldehyde in the final step before
testing stability.

32,805-F

1. A process for stabilizing and flocculating intact or ruptured microbial cells having active enzymes associated therewith which comprises contacting the microbial cells with a partially modified cationic polyelectrolyte.

2. Process of Claim 1 which comprises
(1) encapsulating the flocculated microbial cells with a partially modified cationic polyelectrolyte, and
(2) cross-linking the encapsulated microbial cells.

3. Process of Claim 2 wherein the flocculated microbial cells are cross-linked both before and after encapsulating.

4. The process of any one of Claims 1 to 3 wherein the microbial cells are glucose isomerase- -producing cells.

5. The process of any one of Claims 1 to 4 wherein the partially modified cationic polyelectrolyte is a partially modified polyamine.

6.    The process of any one of Claims 1 to 5 wherein the partially modified cationic polyelectrolyte is a partially carboxyalkylated, or partially phosphonoalkylated, cationic polyelectrolyte wherein the alkyl is $-(CH_2)_n-$ wherein n=1-3, or $-(CHR)-(CH_2)_n-$ wherein R=methyl, ethyl, propyl, or isopropyl, and n=1 or 2.

7.    The process of Claim 6 wherein the partially carboxyalkylated cationic polyelectrolyte is partially carboxymethylated polyethyleneimine.

8.    The process of any one of Claims 1 to 7 wherein the modification of the partially modified cationic polyelectrolyte is from 0.1 to 1.0 equivalents of polyelectrolyte during the flocculating step and 0.2 to 1.0 equivalents of polyelectrolyte during the encapsulating steps.

9.    The process of Claim 8 wherein the partially modified cationic polyelectrolyte is polyethyleneimine which is carboxymethylated in the range of 0.25 to 0.5 equivalents of the imine.

10.    A process for preparing D-fructose from D-glucose which comprises contacting D-glucose with glucose isomerase-producing intact or ruptured microbial cells which have been 1) flocculated with a partially modified cationic polyelectrolyte, and (2) cross-linked.

11.    Process for Claim 10 wherein the microbial cells employed have been encapsulated with a partially modified cationic polyelectrolyte, and again cross-linked.